(19) Europäisches Patentamt
European Patent Office
Office européen des brevets

(11) **EP 1 069 883 B1**

(12) **EUROPEAN PATENT SPECIFICATION**

(45) Date of publication and mention
of the grant of the patent:
**05.10.2011 Bulletin 2011/40**

(51) Int Cl.:
***A61K 8/63*** (2006.01)        ***A61K 8/68*** (2006.01)
***A61Q 19/04*** (2006.01)

(21) Application number: **00905946.0**

(22) Date of filing: **02.02.2000**

(86) International application number:
**PCT/US2000/002750**

(87) International publication number:
**WO 2000/045786 (10.08.2000 Gazette 2000/32)**

(54) **Self-tanning compositions comprising cholesterol sulphate and DHA**

Selbstbräunungszusammensetzungen die Cholesterolsulfat und DHA enthalten

Compositions autobronzantes comprenant du sulfate de cholesterol et de la DHA

(84) Designated Contracting States:
**AT BE CH CY DE DK ES FI FR GB GR IE IT LI LU
MC NL PT SE**

(30) Priority: **08.02.1999 US 246607**

(43) Date of publication of application:
**24.01.2001 Bulletin 2001/04**

(73) Proprietor: **Color Access, Inc.
Melville,
New York 11747 (US)**

(72) Inventors:
• **MAES, Daniel, H.
Huntington, NY 11743 (US)**
• **MARENUS, Kenneth, D.
Dix Hills, NY 11746 (US)**
• **FTHENAKIS, Christina, G.
Dix Hills, NY 11746 (US)**

(74) Representative: **Hirsch & Associés
58, avenue Marceau
75008 Paris (FR)**

(56) References cited:
**EP-A1- 0 386 680        WO-A-90/01323
DE-A- 19 834 812      DE-C- 19 642 872
JP-A- 5 051 314          JP-A- 11 005 742
US-A- 5 459 165**

• **CHEMICAL ABSTRACTS, vol. 132, Columbus,
Ohio, US; abstract no. 170820, WILDEN, WIM VAN
DER ET AL: "A skin-identical lipid concentrate for
an improved skin-barrier function" XP002135991
& FRAGRANCE J. (1999), 27(10), 71-74 , 1999,**

• **CHEMICAL ABSTRACTS, vol. 104, Columbus,
Ohio, US; abstract no. 10400, ABE, TAKASHI ET
AL: "Cosmetics containing cholesteryl sulfates"
XP002135992 & JP 60 161911 A (KANEBO, LTD.,
JAPAN) 23 August 1985 (1985-08-23)**

• **JUNKO SATO ET AL.: THE JOURNAL OF
INVESTIGATIVE DERMATOLOGY, vol. III, no. 2,
August 1998 (1998-08), pages 189-191,**

**Description**

FIELD OF THE INVENTION

The present invention relates to cosmetic compositions. More specifically, the invention relates to topical compositions for artificially tanning the skin containing cholesterol sulphate and methods of using same in treatment of skin. Self-tanning compositions comprising DHA and cholesterol are known from US-A-5459165.

BACKGROUND OF THE INVENTION

The stratum corneum represents the major chemical and physical barrier between the body and the environment. It is formed by a process in the epidermis which involves the transformation of germinative cells into terminally differentiated cells; the process of transformation takes approximately one month, by which time the terminally differentiated cells are shed from the skin surface. The cells at the outermost layer of the skin, which are constantly being sloughed off, are replaced by cells that are generated by the mitotic activity of the basal layer of the epidermis. In the course of their migration from the basal layers to the upper levels of the skin, these cells produce and accumulate keratin, to the point at which there is virtually no cytoplasm remaining; the cell then dies and is shed, to be followed by another phalanx of migrating epidermal cells. The thickness of the stratum corneum and epidermis in general varies in different regions of the body.

This cornified barrier performs a number of functions. A particularly important aspect of its presence is as a physical barrier, between the deeper layers of the skin as well as the internal organs and the environment. Prevention or attenuation of penetration of UV radiation, as well as other harmful stimuli such as free radicals, to the deeper skin layers is one very critical aspect of this skin layer. Unfortunately, as with many other skin functions, the performance capacity of the stratum corneum becomes progressively diminished with age. The turnover rate of the stratum corneum is considerably decreased in older individuals, and the cornified layer gradually becomes much thinner, thereby reducing the efficacy of this physical barrier and permitting easier penetration of harmful stimuli such as UV rays. This in turn leads to UV-damage to the dermal layers of the skin, resulting in degradation of collagen and elastin, finally resulting in wrinkling and skin atrophy. Moreover, the thinning of the stratum corneum can result in a greater visibility of the wrinkling and atrophy, the cause of which is rooted in the dermis.

Notwithstanding the obvious importance of the stratum corneum in maintaining a healthy youthful appearance of the skin, rehabilitation and maintenance of the dermis has been a major cosmetic focus in preventing the appearance of aging; relatively little attention has been paid to developing cosmetic means for maintaining a fairly consistent level of stratum corneum function into old age.

Summary of the Invention

The invention relates to a composition for artificially tanning the skin and prolonging retention of tanned skin cells comprising cholesterol sulphate and at least one self-tanning agent wherein the cholesterol sulphate is present in an amount in the range of 0.05% to 10%, and the self-tanning agent is present in the composition in an amount in the range of 2.5 to 10% when it is DHA, and in an amount of 1-10% when it also contains imidazole, wherein said amounts are by weight of the total composition.

In yet another embodiment, the invention provides a method for artificially tanning and prolonging retention of tanned skin cells comprising applying to the skin the composition of the invention.

Brief Description of the Figures

Figure 1 illustrates the condition of stratum corneum thickness under different cholesterol sulphate treatment regimens, as described in Example I: (A)control (no treatment) ; (B) 1% ethanol vehicle control; (C)cholesterol sulphate, 0.01$\mu$g/ml; (D) cholesterol sulphate, 1$\mu$g/ml; (E)cholesterol sulphate, 1$\mu$g/ml; (F) cholesterol sulphate 10$\mu$g/ml.
Figure 2 illustrates the duration of the self-tanning action of DHA with and without cholesterol sulphate.
Figure 3 illustrates the duration of the self-tanning action of DHA with and without cholesterol sulphate and a lipid mix.

Detailed Description of the Invention

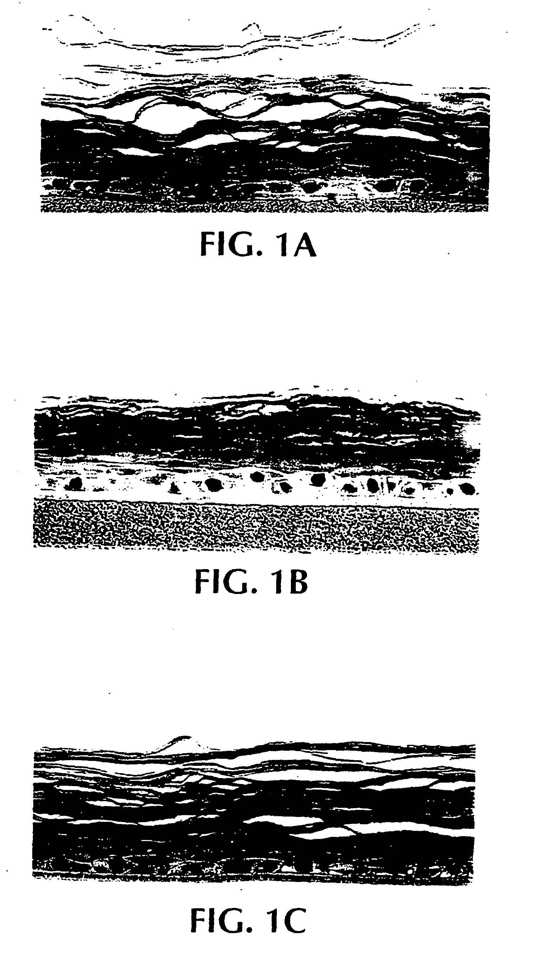

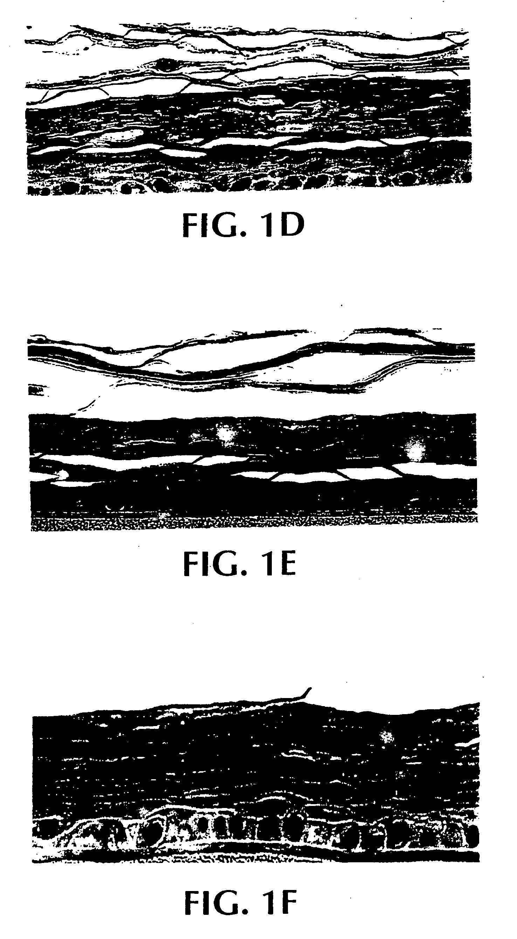

The present invention, in its various embodiments, is predicated on the observation that cholesterol sulphate, when applied topically to the skin, enhances the cohesion of the stratum corneum resulting in a more prolonged retention of the layers of the stratum corneum. Specifically, it has been observed that application of cholesterol sulphate to skin cells results in a distinct, dose-dependent, increase in the thickness of the layers of the stratum corneum, as shown in Figures 1C-F. The observation is important for a number of different applications; a particularly significant application is in the maintenance of the texture of older skin. A current common means of enhancing smoothness of the skin is to encourage exfoliation. However, exfoliation necessarily involves a high rate of turnover of the stratum corneum, and consequent thinning of this layer of the skin. While not an issue in youthful skin, desquamation in older skin can, in some cases, simply exacerbate a problem already established, namely, the natural thinning observed with age. Thus, application of cholesterol sulphate to retard desquamation and maintain stratum corneum thickness represents an entirely new direction in the treatment and maintenance of older, thinning skin. A thicker stratum corneum aids in preventing or retarding the appearance of fine lines and wrinkles which so frequently characterize thinning skin. At the same time, the enhanced cohesion of the stratum corneum results in an effective strengthening of the protective lipid barrier naturally provided therein.

To achieve this effect, the cholesterol sulphate or salts thereof can be applied in any type of cosmetically or pharmaceutically acceptable vehicle for topical application with which the active component is compatible, e.g., a gel, a cream, a lotion, an ointment, a mousse, a spray, a solid stick, a powder, a suspension, a dispersion, and the like. Preferably, however, the cholesterol sulphate is not provided in a liposome formulation, and is formulated in a composition containing relatively low levels of emulsifiers. Techniques for formulation of various types of vehicles are well known to those skilled in the art, and can be found, for example, in Chemistry and Technology of the Cosmetics and Toiletries Industry, Williams and Schmitt, eds., Blackie Academic and Professional, Second Edition, 1996, and Remington's Pharmaceutical Sciences, 18th Edition, 1990, the contents of which are incorporated herein by reference. Cholesterol sulphate is effective in the claimed function when provided in the composition in an amount of from 0.05 to 10%, preferably from 0.5 to 5%, most preferably 1 to 3%, all by weight of the total composition.

The thickening and cohesion of the stratum corneum also provides other benefits, which, in certain specific applications, can be appreciated by individuals of all ages. The stratum corneum represents an important physical barrier between the environment and the deeper skin layers as well as the internal organs. The presence of this thicker layer thus will provide a greater level of protection than is possible with a loose, flaking stratum corneum. Although this property can be exploited in a number of ways, perhaps the most important is the enhanced self-protection from UV rays. The thicker stratum corneum means an increase in the Minimal Erythemal Dose of UV which will result in sunburn or more serious skin damage. In connection with this aspect cholesterol sulfate may be beneficially combined with one or more sunscreens for an enhanced UV protective composition which provides both short- and long-term protection.

The cholesterol sulphate is used according to the invention in the enhancement and prolongation of self-tanning products. One of the recognized limitations of self-tanners, which are normally based on dihydroxyacetone (DHA) as the active component, is that the tan on the skin lasts only as long as the skin cells receiving the DHA remain in place. In the normal course of events, then, a self-applied tan usually lasts no more than 5 days, i.e., for as long as it takes for the stratum corneum layer to which the DHA was applied to fully turn over. When cholesterol sulphate is combined with DHA, or any other self-tanning agent, in a typical self-tanning formulation, however, the rate of turnover of the stratum corneum to which the composition is applied is slowed down, thereby permitting a longer rate of retention of the "tanned" cells, and thus prolonging the length of time the tan remains visible on the skin. Thus, the invention provides a self-tanning composition comprising from 0,05% to 10% of cholesterol sulphate and an effective amount of a self-tanning agent. The self-tanner is DHA, which is applied in an amount of from 2.5 to 10% by weight of the formulation. The self-tanner may also be imidazole, in combination with DHA, in an amount of about 1-10%, preferably about 1.5-7.5%.

In addition to its use in therapeutic products, cholesterol sulfate can also be beneficially added to color cosmetic products. In this regard, effective amounts of cholesterol sulphate can be added to makeup formulations such as foundations, blushes, lipsticks and glosses, eyeliners, eyeshadows, and the like. A particular advantage may be obtained with such formulations, in that the retardation of desquamation may enhance makeup retention on the skin to which it is applied. The sunscreen/cholesterol sulfate combination may also be effectively employed in such products.

In all formulations in which cholesterol sulphate is employed, it is preferred that the cholesterol sulphate be combined with other components of the naturally occurring lipid barrier. In a particularly preferred embodiment of the artificially tanning composition the cholesterol sulphate is combined with at least one of each of fatty acids, ceramides, and a sterol, preferably cholesterol. Fatty acids may be up to 24 carbon atoms in length. Examples of preferred fatty acids include butyric acid, caproic acid, octanoic acid, decanoic acid, dodecanoic acid, tetradecanoic acid, palmitic acid, stearic acid, linoleic acid and oleic acid. Particularly preferred are fatty acids with a $C_{12}$ to $C_{20}$ chain length.

The ceramides to be employed in the compositions of the invention are sphingolipids, having a sphingosine or related molecule backbone with fatty acids or ω-esterified fatty acids linked to an amino group on the sphingosine, and in some cases, with saccharide moieties linked to the terminal hydroxyl of the sphingosine. In particular, the compositions may contain ω-esterified ceramides or acylceramides, cerebrosides, ω-esterified cerebrosides, or acylglycosyl sphingolipids. Particularly preferred types of ceramides for the present compositions are ceramide III and cerebrosides.

In those compositions in which cholesterol sulphate is combined with these lipids, the lipid components each can be used in an amount of from about 0.05 to 10%, preferably 0.5 to about 5%, most preferably about 1 to about 3%, all by weight of the total composition. The cholesterol sulfate and the lipid components can be present in substantially equal amounts in the composition. It will be understood from the foregoing that the lipid component need not be pure lipid, but rather may be natural extracts containing one or more desirable lipids, and used in amounts consistent with attaining the concentrations recommended above.

The compositions of the invention are applied to the skin in a manner appropriate to the intended end result. For example, for the general promotion of the appearance of young, healthy skin by retardation of desquamation and maintenance of stratum corneum, the the best results are achieved after regular application over a period of time. A preferred method of obtaining the benefits of the composition is via chronic topical application of a safe and effective amount of a composition containing cholesterol sulphate. It is suggested as an example that topical application of the composition, in an amount of from about 0.1 mg/cm$^2$ to 2 mg/cm$^2$ of skin, be performed from about once per week to about 4 or 5 times daily, preferably from about 3 times a week to about 3 times daily, most preferably about once or twice per day. By "chronic" application, it is meant herein that the period of topical application may be over the lifetime of the user, preferably for a period of at least about one month, more preferably from about three months to about twenty years, more preferably from about six months to about ten years, more preferably still from about one year to about five years, thereby resulting in the treatment or prevention of the external signs of photo- or chronoaging.

The composition is applied in similar amounts, on the portion of the skin to be tanned, with repetition, again, on an as-needed basis.

The invention is further illustrated by the following non limiting examples:

Example I

This example illustrates the ability of cholesterol sulphate to retard desquamation and maintain stratum corneum thickness.

Matek skin equivalents are obtained and prepared for use in accordance with the supplier's protocol. Equilibrated skins are treated topically with dilutions of cholesterol sulphate. Cholesterol sulphate is solubilized 1 mg/ml in ethanol, and serially diluted 10-fold to yield doses of 0.01, .1, 1 and 10$\mu$g/ml. Each dose is added topically to an equivalent, using a 100$\mu$l volume. Treatment is repeated daily along with media replacement over a three day period. One sample is treated with 1% ethanol, representing a vehicle control. Following treatment, equivalents are fixed according to standard protocol, and sent for histological preparations. Figures 1A-F show stained sections of the two controls plus the treatment samples. The figures show a very loose organization of the stratum corneum in the media control, with a gradual increase in organization and cohesion of the stratum corneum seen in the treatment samples, which increases with the amount of cholesterol sulphate in the treatment. Some compaction is seen in the ethanol treated sample, which is believed due to dehydration of the sample combined with lipid removal.

Example II

The following is a composition according to the present invention:

| Material | Weight % |
| --- | --- |
| Phase I | |
| isocetyl alcohol | 2.5 |
| octyl hydroxystearate | 2.0 |
| alpha hydroxylauric acid | 0.5 |
| Phase II | |
| purified water | QS |
| cyclodextrin | 1.0 |
| Phase III | |
| ethoxydiglycol | 5.0 |
| laureth-23 | 1.5 |
| dipropylene glycol | 1.0 |
| sodium hyaluronate (1%) | 1.2 |

(continued)

| | |
|---|---|
| Phase III | |
| pantethine | 0.1 |
| | |
| PhaseIV | |
| sucrose | 2.0 |
| dihydroxyacetone | 5.0 |
| | |
| Phase V | |
| cyclomethicone | 12.0 |
| dimethicone | 3.0 |
| cyclomethicone/dimethicone | 2.0 |
| tricaprylyl citrate | 1.5 |
| dimethicone | 3.0 |
| | |
| Phase VI | |
| malvaceae extract | 0.2 |
| fragrance | 0.4 |
| tocopheryl acetate | 0.1 |
| wheat bran extract | 0.2 |
| linoleic acid | 0.2 |
| sodium cholesterol sulphate | 0.2 |
| | |
| Phase VII | |
| nylon-12 | 2.0 |
| | |
| Phase VIII | |
| polyquaternium-37/propylene glycol | 1.2 |

Example III

A study is conducted to determine the effect of cholesterol sulphate on skin flakiness, as an indicator of its effect in reducing desquamation. Fifteen subjects between the ages of 21 and 65 years are selected for the study. The subjects report for the study without moisturizers or any other products on their hands and their baseline measurements are taken. The subjects are given a product containing 0.5% cholesterol sulphate in a water and oil emulsion base to take home and self-administer on their right hands only, twice a day in the morning after washing and in the evening at least 15 minutes before bedtime for four weeks. The left hand serves as the untreated control site. The subjects are only allowed to use the test product and specifically log its use in a daily diary. At the end of two and four weeks the subjects return for testing without applying the product for at least 12 hours and they are re-evaluated under the same conditions.
Evaluation of flakiness is determined via the D-Squame Discs Method and Image Analysis. Briefly, four D-Squame discs are firmly pressed on the back of each hand with hand held uniform pressure device and removed by gently pulling away from the skin. The D-Squame discs are mounted on clear microscope slides and labeled according to subject's name and visit. Desquamation is evaluated from the D-Squame discs via the image analyzer. Skin evaluation is carried out before treatment, and after two and four weeks of treatment.
An OPTIMA image analyzer is used to evaluate skin flakiness. The D-Squame samples containing the stratum corneocytes are placed under a camera on top of a light table and each image is imported into the image analyzer. The average Gray Value corresponding to the sample density is measured. The denser the sample, the higher the Gray Value difference. The treated skin shows a 22.5% decrease in flakiness relative to baseline after two weeks, and a 24.1% decrease after 4 weeks. The decrease in flakiness is apparently due to the observed effect on cohesion of the stratum corneum.

Example IV

This example illustrates the efficacy of the addition of cholesterol sulphate to DHA in enhancing duration of self-tanning. Two products are prepared for testing, one a control formulation containing 5% DHA, and the second the test formulation containing 5% DHA, and 0.2% sodium cholesterol sulphate. A total of 10 panelists participate in the study. The control formulation is applied to the right arm and the test formulation on the other. Equal amounts of the products (800μl) are dispensed and blended in until absorbed.

Color measurements are obtained with a Chromameter before treatment, 24 hours after treatment, and 4 days and 5 days. Decrease in reflectance and increase in red coloration and yellow coloration (ΔL*, Δa*, Δb*) obtained from the Chromameter are calculated as compare to baseline skin color. Total color change ΔE* is calculated for each time point as follows:

$$\Delta E^* = \text{square root of } (\Delta L^*)^2 + (\Delta a^*)^2 + (\Delta b^*)^2$$

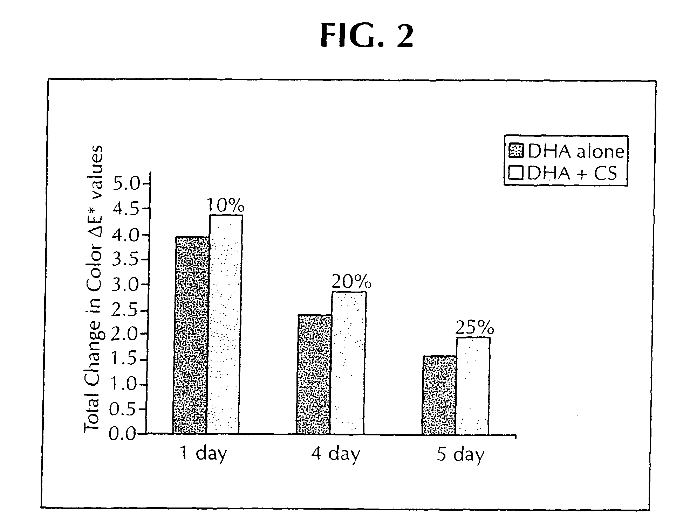

The results, shown in Figure 2, demonstrate that there is a decrease in skin reflectance and an increase in skin redness and yellow coloration due to the self-tanning effect of the products. Total change in color values (ΔE*) observed from the graph show that there is 10% darker color on the arms treated with the formulation containing the cholesterol sulphate as compared with the one treated with DHA alone. After 4 and 5 days, there is still 20% and 25% darker tan on the site treated with DHA and cholesterol sulphate, as compared with the site treated with DHA alone. These data show that the addition of cholesterol sulphate to DHA results in a longer lasting tan.

Example V

This Example illustrates the efficacy of a composition containing DHA combined with cholesterol sulphate and a lipid mix in enhancing the intensity and duration of self-tanning.

Two products are tested: a test product containing 5% DHA, 0.2% sodium cholesterol sulphate, 0.2% linoleic acid and 0.2% SC complex, containing wheat bran extract and olive oil extract, and a standard self-tanning product containing only 5% DHA as control.

A total of 22 panelists participate in the study, divided into two groups of eleven each. The control is applied on the right arm, and the test product is applied on the left arm. Equal amounts of the product (800μl) are dispensed and blended in until absorbed.

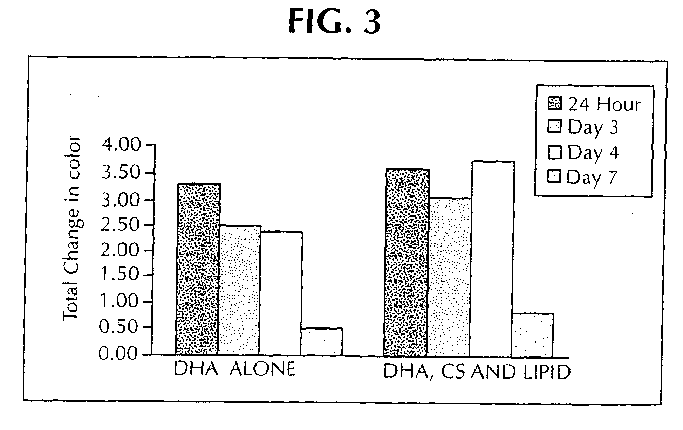

Color measurements are taken as described in Example IV. The results, shown in Figure 3, demonstrate that there is a decrease in skin reflectance and an increase in skin redness and yellow coloration due to the self-tanning effect of the products. The 5% DHA product with the lipid mix appears to retain color more efficiently during the entire seven-day study period when compared with the control product.

**Claims**

1. A composition for artificially tanning the skin and prolonging retention of tanned skin cells comprising cholesterol sulphate and at least one self-tanning agent wherein the cholesterol sulphate is present in an amount in the range of 0.05% to 10%, and the self-tanning agent is present in the composition in an amount in the range of 2.5% to 10% when it is DHA and in an amount of about 1-10% when it also contains imidazole, wherein said amounts are by weight of the total composition.

2. The composition of claim 1 in which the composition comprises 1% to 3% cholesterol sulphate.

3. The composition of any one of claims 1 or 2 comprising from 0.05% to 10% of at least one of each of fatty acids, ceramides and a sterol.

4. The composition of claim 3 in which the fatty acid is a $C_{12}$-$C_{20}$ fatty acid.

5. The composition of claim 3 or 4 in which the ceramide is ceramide III or a cerebroside.

6. The composition of any one of claims claim 3 to 5 which comprises cholesterol sulphate, a $C_{12}$-$C_{20}$ fatty acid, ceramide III or a cerebroside, and cholesterol.

7. A method for artificially tanning the skin and prolonging retention of tanned skin cells comprising applying to the skin the composition of any one of the claims 1 to 6.

**8.** The method of claim 7 for retarding desquamation and enhancing the thickness of the stratum corneum.

**Patentansprüche**

**1.** Zusammensetzung zum künstlichen Bräunen der Haut und zum Verlängern der Bewahrung gebräunter Hautzellen, Cholesterinsulfat und mindestens ein Selbstbräunungsmittel umfassend, wobei das Cholesterinsulfat in einer Menge im Bereich von 0,05 % bis 10 % vorhanden ist und das Selbstbräunungsmittel in der Zusammensetzung in einer Menge im Bereich von 2,5 % bis 10 % vorhanden ist, wenn es DHA ist, und in einer Menge von etwa 1 bis 10 %, wenn es außerdem Imidazol enthält, wobei sich die Mengen auf das Gewicht der Gesamtzusammensetzung beziehen.

**2.** Zusammensetzung nach Anspruch 1, wobei die Zusammensetzung 1 % bis 3 % Cholesterinsulfat umfasst.

**3.** Zusammensetzung nach einem der Ansprüche 1 oder 2, zu 0,05 % bis 10 % mindestens jeweils eine Fettsäure, ein Ceramid und ein Sterin umfassend.

**4.** Zusammensetzung nach Anspruch 3, wobei die Fettsäure eine $C_{12}$-$C_{20}$-Fettsäure ist.

**5.** Zusammensetzung nach Anspruch 3 oder 4, wobei das Ceramid Ceramid III oder ein Cerebrosid ist.

**6.** Zusammensetzung nach einem der Ansprüche 3 bis 5, die Cholesterinsulfat, eine $C_{12}$-$C_{20}$-Fettsäure, Ceramid III oder ein Cerebrosid und Cholesterin umfasst.

**7.** Verfahren zum künstlichen Bräunen der Haut und zum Verlängern der Bewahrung gebräunter Hautzellen, das Aufbringen der Zusammensetzung nach einem der Ansprüche 1 bis 6 auf die Haut umfassend.

**8.** Verfahren nach Anspruch 7 zum Verzögern der Desquamation und zum Erhöhen der Dicke des Stratum corneum.

**Revendications**

**1.** Une composition pour bronzer artificiellement la peau et prolonger la rétention des cellules de peau bronzée, comprenant du sulfate de cholestérol et au moins un agent autobronzant, dans laquelle le sulfate de cholestérol est présent en une quantité de 0,05% à 10%, et l'agent autobronzant est présent dans la composition en une quantité de 2,5% à 10% lorsqu'il consiste en DHA et en une quantité de 1 à 10% quand il contient également de l'imidazole, lesdites quantités étant exprimées par rapport au poids total de la composition.

**2.** La composition selon la revendication 1, dans laquelle la composition comprend de 1% à 3% de sulfate de cholestérol.

**3.** La composition selon une quelconque des revendications 1 ou 2, comprenant de 0,05% à 10% d'au moins un acide gras, d'au moins une céramide et d'au moins un stérol.

**4.** La composition selon la revendication 3, dans laquelle l'acide gras est un acide gras en $C_{12}$-$C_{20}$.

**5.** La composition selon la revendication 3 ou 4, dans laquelle la céramide consiste en céramide III ou en cérébroside.

**6.** La composition selon une quelconque des revendication 3 à 5, qui comprend du sulfate de cholestérol, un acide gras en $C_{12}$-$C_{20}$, un céramide III ou un cérébroside, et du cholestérol.

**7.** Un procédé pour bronzer artificiellement la peau et prolonger la rétention des cellules de peau bronzée comprenant l'application à la peau de la composition selon une quelconque des revendications 1 à 6.

**8.** Le procédé selon la revendication 7, pour retarder la desquamation et augmenter l'épaisseur du stratum corneum.

FIG. 1A

FIG. 1B

FIG. 1C

FIG. 1D

FIG. 1E

FIG. 1F

# FIG. 2

# FIG. 3

**EP 1 069 883 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5459165 A

### Non-patent literature cited in the description

- Chemistry and Technology of the Cosmetics and Toiletries Industry. Blackie Academic and Professional, 1996

- Remington's Pharmaceutical Sciences. 1990